# EUROPEAN PATENT APPLICATION

(11) **EP 0 802 140 A2**
(43) Date of publication of application: **22.10.1997**
(21) Application number: 97104663.6
(22) Date of filing: 19.03.1997
(51) Int. Cl.: B65H 23/00, B65H 39/00, B32B 31/00, A61F 13/15

(54) **Method of assembling web or film materials utilising a static electrical charge**

(30) Priority: 22.03.1996 US 620517
(71) Applicant: THE PROCTER & GAMBLE COMPANY, Cincinnati, Ohio 45202 (US)
(72) Inventor: Duempelmann, Dirk, Dr.-Ing., 53909 Zuelpich (DE)
(74) Representative: Canonici, Jean-Jacques

(57) **Abstract**

The present invention relates to a method of assembling web or film materials during a continuous process, wherein the method relies on the use of a static electrical charge. In particular, the method is applied to the assembly of web or film materials (10,11) during the process of making disposable absorbent articles such as female hygiene products, diapers, incontinence products, bandages, and the like.

## Description

### Field of the invention

The present invention relates to a method of assembling web or film materials during a continuous process, wherein the method relies on the use of a static electrical charge. In particular, the method is applied to the assembly of web or film materials during the process of making disposable absorbent articles such as female hygiene products, diapers, incontinence products, bandages, and the like.

### Background of the invention

A multiplicity of apparatus is currently available for assembling materials, in particular for receiving strips of material on a rotating drum and transferring same to a moving surface of a transfer roll. Typically, the strips of material are held in position on a rotating drum by a vacuum effect before the transfer takes place. The vacuum effect is created by drawing air through perforations or holes that are present on the outer surface of the rotating drum. The prior art is rich with examples of such devices.

EP 0 577 241 describes a method and apparatus for handling linerless label material. The apparatus includes such elements as a non-stick circumferential surface feed roll and a hardened vacuum anvil cylinder co-operating with a cutting cylinder having a radially extending knife blade. A vacuum is required in order to insure that the labels stay in place until it is desired to remove them. For this purpose, a vacuum chamber is provided. More specifically, the vacuum chamber is connected up to a vacuum pump, which pulls air through the spaces between the conveyor tapes, thereby providing a force, which holds the labels on the conveyor tapes. The conveyor tapes transport the labels in the transport direction. Typically, the elements to which the labels are applied may comprise moving envelopes.

EP 0 219 114 relates to an applicator for applying two or more tapes to a moving web and, more particularly, to tape tabs which are used in diapers and incontinence pads. Once a discrete segment of continuous web has been severed between the anvil and knife rolls, the transversely arranged vacuum holes in the surface of the vacuumised transfer roll hold the cut segment firmly against the transfer roll surface. The invention relies on the use of a non-porous continuous web material.

EP 0 450 650 relates to an apparatus and process for receiving and rotating strips of material on a transfer roll towards a moving surface and transferring the strips to the moving surface. The strips of material are held on their respective transferring elements of the vacuum anvil roll by a vacuum effect created through holes in the outer surface of the transferring element.

Nevertheless, it is currently widely recognised and accepted that systems incorporating vacuum devices present barriers to the use of materials with high air throughputs, thereby limiting extensively the possibility of creating breathable parts for disposable absorbent articles, which are known to satisfy the genuine consumer need for dryness, comfort and healthier skin. Such vacuum devices can result in undesirable pleats, wrinkles or even tearing in the material and can lead to skewed alignment of the material with the moving surface of the transfer roll. In addition, there is a need to provide ancillary equipment in order to vary the perforation/hole pattern on the vacuum devices to cope with different lengths and widths of material. Furthermore, the build-up of fibre and dust in the vacuum chamber generated by the cutting process can affect machine efficiency and lead to forced stoppages along the line for mechanical servicing.

Therefore, it has been recognised by those skilled in the art that it would be extremely desirable to develop a system for assembling materials that does not require the use of a vacuum device. The co-pending patent application WO 96/28959 describes an apparatus and method for applying an electrostatic force to a moving web of non-metallic material in conjunction with a web processing machine, which applies a coating to the web. The web is supplied from a storage drum and moved along a pre-determined path to be taken up and stored on a take-up drum. In the processing zone, a coating system applies a coating to a surface of the web. The application of a controlled electrostatic force provides improved control of the web speed and temperature, and also results in a more consistent and uniform coating and curing of the web. The document, however, does not discuss the notion of assembling web or film materials wherein one of the web or film materials to be transferred is held in position on a rotating drum using a static electrical charge before being transferred to another web or film material on a rotating transfer roll.

Thus, the man skilled in the art has realised that the benefits of the present invention range from a system capable of handling in an extremely effective manner high air throughput web or film materials, in addition to non-porous web or film materials; to a system that is not affected by the fibre and dust contamination arising from the cutting process; to a rotating drum of simpler construction; to a system requiring a lower power consumption, i.e., a reduction from 1000 watts for the vacuum device such as a vacuum pump to 100 watts for the electrostatic charging unit; to the elimination of ancillary equipment for varying the lengths and widths of the web or film materials to be applied; and to a system that is characterised by a lower noise level, which is greatly valued by the machine operators and which provides for a more comfortable working environment.

### Summary of the invention

The present invention relates to a method of assembling web or film materials during a continuous process. The method comprises the steps of: (a) providing a first web or film material from a first supply assembly; (b) providing a second web or film material from a second supply assembly; (c) transferring the first web or film material to the second web or film material on a transfer roll; and before step (c), the method further comprises the step of imparting a static electrical charge to hold the first web or film material in position on a rotating drum.

In a preferred embodiment of the present invention, the first web or film material is cut into pieces for application to the second web or film material before a static electrical charge is imparted. In a further embodiment of the present invention, the first web or film material is cut into pieces for application to the second web or film material after a static electrical charge is imparted.

In a preferred embodiment of the present invention, the static electrical charge is imparted to the first web or film material. In order to transfer the first web or film material to the second web or film material, the static electrical charge imparted to the first web or film material is opposite in polarity to that of the second web or film material. The first web or film material and/or the second web or film material can be discharged upon unwinding from their respective supply assemblies to give a defined zero charge before the static electrical charge is imparted. This step ensures effective static electrical charging. In yet another preferred embodiment of the present invention, the static electrical charge is imparted to the rotating drum.

According to the teachings of the present invention, the first web or film material can be provided in the form of either an adhesive coated layer of web or film material or a non-adhesive coated layer of web or film material. In addition, the first and/or the second web or film materials are/is provided in the form of porous web or film materials.

The method of the present invention is directed to the making of disposable absorbent articles such as feminine hygiene products, diapers, incontinence products, bandages, and the like. In particular, the application of the method to a sanitary napkin is described.

### Brief description of the drawings

It is believed that the invention will be better understood from the foregoing description in conjunction with the accompanying drawings in which:
Figure 1 is a schematic view of a method of assembling web or film materials according to the teachings of the present invention;
Figure 2 is a plan view of a sanitary napkin incorporating the method as illustrated in Figure 1; and
Figure 3 is a schematic top plan view of the main body portion of the sanitary napkin as shown in Figure 2.

### Detailed description of the invention

As intended herein, the term "disposable" describes absorbent articles that are not intended to be laundered or otherwise restored or reused as an absorbent article (i.e., they are intended to be discarded after a single use and, preferably, to be recycled, composted or otherwise disposed of in an environmentally compatible manner). It should be understood that the method of the present invention is applicable to the manufacture of disposable absorbent articles such as diapers, training pants, incontinence products and briefs, feminine hygiene products and garments, bandages, and the like. Nevertheless, the method is not limited to the these types or configurations of disposable absorbent articles.

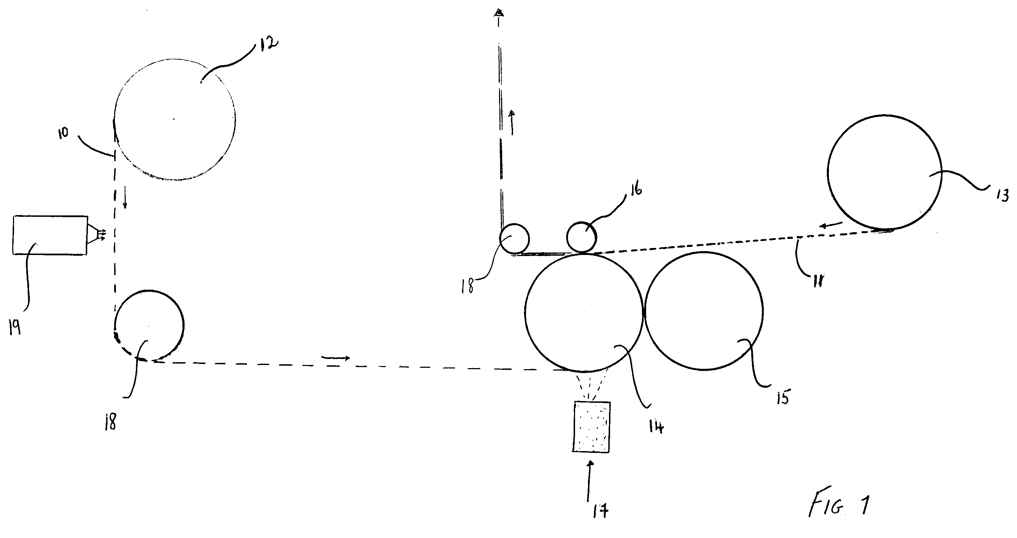

Reference is made to Figure 1 for a schematic explanation of the method according to the present invention for assembling web or film materials during a continuous process. For clarity purposes, the downstream destination of the material structure formed from the present invention is not shown. The schematic figure shows, in particular, a first supply assembly 12, a second supply assembly 13, a rotating drum 14 which in this instance is a rotating anvil roll 14, a rotating knife roll 15, a transfer roll 16 and a electrostatic charging unit 17. For simplicity, the apparatus for the method described herein is considered to comprise rolls 12, 13, 14, 15, 16. It is not, however, intended in any way to limit the invention to an apparatus comprising rolls per se.

The basic steps in the method of assembling web or film materials during a continuous process according to the present invention comprise the steps of (a) providing a first web or film material 10 from a first supply assembly 12; (b) providing a second web or film material 11 from a second supply assembly 13; (c) transferring the first web or film material 10 to the second web or film material 11 on a transfer roll; and before step (c), the method further comprises the step of imparting a static electrical charge to hold the first web or film material 10 in position on a rotating drum 14.

With regard to steps (a) and (b), it is well known to provide materials in the form of webs or films in roll form and to provide unwinding and splicing means to enable the forwarding of continuous lengths of such web or film materials through means to make material structures for use in disposable absorbent articles. A first web or film material 10 is supplied from the first supply assembly 12; the web or film material being provided in the form of a continuous layer. A second web or film material 11 is supplied from the second supply assembly 13; the web or film material being also provided in the form of a continuous layer. As used herein, the term "layer" does not necessarily limit the web or film to a single stratum of material. According to the present invention, the first web or film material 10 and the second web or film material 11 can be provided in the form of either porous - air permeable, fluid permeable or vapour permeable - or non-porous web or film materials. Furthermore, the first web or film material 10 and the second web or film material 11 used in the assembling method can be similar in composition or different.

At least one guide roll 18 guides and advances the first web or film material 10 from the first supply assembly 12 to the rotating anvil roll 14. The second web or film material 11 is advanced towards the transfer roll 16 with or without the aid of guide rolls. At least one other guide roll 18 advances the material structure from the transfer roll 16 towards its downstream destination. Means for driving the rolls 12, 13, 14, 15, 16 and means for the guide rolls 18 are not shown. In a preferred embodiment of the present invention, the first web or film material 10 can be provided in the form of a coated layer of web or film material, preferably an adhesive coated layer of web or film material. The coating is typically released from a coating device 19 to contact at least one surface of the first web or film material 10. The coating can be either an adhesive (preferred option), an ink or an agent designed to treat the first web or film material 10. The coating device 19 is generally situated upstream of the first supply assembly 12. In another preferred embodiment of the present invention, the first web or film material 10 can be provided in the form of an uncoated layer of web or film material, preferably a non-adhesive coated layer of web or film material.

The drive shaft of the transfer roll 16 is rotated to cause rotation of the transfer roll 16 and transfer roll gear, which is in meshing engagement with the anvil gear. The anvil gear rotates the anvil drive shaft, which in turn rotates the anvil roll 14. The anvil gear is in meshing engagement with the knife gear to rotate the knife drive shaft, which in turn rotates the knife roll 15. By varying the diameter of the gears, the rotational speeds of the knife roll 15 and anvil roll 14 can be varied relative to each other and to the transfer roll 16, as desired. Typically, the anvil roll 14 and the knife roll 15 have the same surface speed as the second web or film material 11; the surface speed of the first web or film material 10 being lower. This lower speed enables the knife blade of the knife roll 15 to cut the first web or film material 10 into pieces. The knife roll 15 includes at least one knife blade and the anvil roll 14 includes at least one blade cutting surface for cutting the first web or film material 10 into pieces. Typically, the number of blades and cutting surfaces depend on the selected length for the pieces. In another embodiment of the present invention, the method comprises the step of cutting the first web or film material 10 into pieces for application to the second web or film material 11 on the transfer roll 16 after the static electrical charge has been imparted. The pieces of web or film material still remain attached to the anvil roll 14 as a result of the static electrical charge remaining in the pieces for a certain duration of time. In an even further embodiment of the present invention, the method comprises the step of cutting the first web or film material 10 into pieces for application to the second web or film material 11 before the static electrical charge is imparted. According to this embodiment, the pieces of first web or film material 10 can be held in position by other means. Thereafter, the pieces can be charged and held in position on another rotating drum with the aid of a static electrical charge before being transferred to the second web or film material 11 on the transfer roll 16.

The portion of the method that is of primary interest is the electrostatic charging process, which uses a static electrical charge to hold the first web or film material 10 in position on a rotating drum, or on the rotating anvil roll 14. The electrostatic charging unit 17 can comprise any suitable apparatus for imparting a static electrical charge. Typically, the electrostatic charging unit 17 comprises a power source, at least one electrostatic generating charging bar and wires to connect the electrostatic generating charging bar to the power source. Nevertheless, the electrostatic charging unit 17 may also comprise an electrode; a brush of conducting material such as a carbon fibre brush or a metallic brush; or the electrostatic charging unit 17 can generate ionised air. In the preferred embodiment of the method of the present invention, the electrostatic generating charging bar comprises a SIMCO HD-C charging bar with 475 millimetres total length and 400 millimetres active length and the power source comprises a SIMCO CH-25 electrostatic generating equipment power supply, obtained from the SIMCO Company Inc. of Hatfield, PA, a subsidiary of Illinois Tool Works Company of Glenview, IL, or equivalent. The electrostatic generating charging bar is parallelepipedly shaped although this is not limiting and it may have its longitudinal axis oriented in the cross machine direction or in the machine direction, preferably in the cross machine direction. Typically, relatively high voltages (in the range of between about 10,000 to 50,000 DC volts, and preferably about 20,000 DC volts) are needed to induce charges to hold the first web or film material 10 in position on the rotating anvil roll 14. Nevertheless, very little amperage is generally required (approximately 2 milliamps). Two or more electrostatic generating charging bars may be used, placed one after the other, it the web or film material 10 in use has the tendency to discharge quickly.

The charging bar is typically installed below the anvil roll 14 and generates ions of one polarity that are accelerated towards either the first web or film material 10 or the anvil roll 14. In a preferred embodiment of the present invention, the electrostatic charging unit 17 imparts a static electrical charge (either positive or negative) to the first web or film material 10. The charge makes the first web or film material 10 adhere to the grounded metallic surface of the rotating anvil roll 14. In another preferred embodiment of the present invention, the electrostatic charging unit 17 imparts a static electrical charge (either positive or negative) to the rotating anvil roll 14. For this embodiment, the attractive force between the first web or film material 10 and anvil roll 14 tends to be higher.

In certain cases, a charge can be generated in a web or film material simply by unwinding it due to friction, for example. If the charge has a polarity opposite to that of the charge applied by the electrostatic charging unit 17, then both charges will cancel each other out and no effect of charging will be observed. Therefore, as a precautionary measure, the first web or film material 10 and/or the second web or film material 11 are/is discharged on unwinding from the first supply assembly 12 and/or the second supply assembly 13 before being charged.

As described above, the first web or film material 10 can be provided in the form of a coated or an uncoated layer of web or film material. For the case of the coated web or film material, the adhesive on the back surface of the web or film material 10 permits the web or film material 10 to be transferred readily and easily to the second web or film material 11 on the transfer roll 16. For transfer purposes, the first web or film material 10 is in the form of pieces. For the case of the uncoated web or film material, a static electrical charge can be employed to transfer the first web or film material 10 to the second web or film material 11 on the transfer roll 16. The static electrical charge imparted to the first web or film material 10 is opposite in polarity to the charge imparted to the second web or film material 11. The first web or film material 10 will thus be transferred to the second web or film material 11 on the transfer roll 16 since the force pulling it towards the second web or film material 11 is greater than the force pulling it towards the rotating anvil roll 14. The first web or film material 10 will remain temporarily attached to the second web or film material 11 until both the first web or film material 10 and the second web or film material 11 discharge.

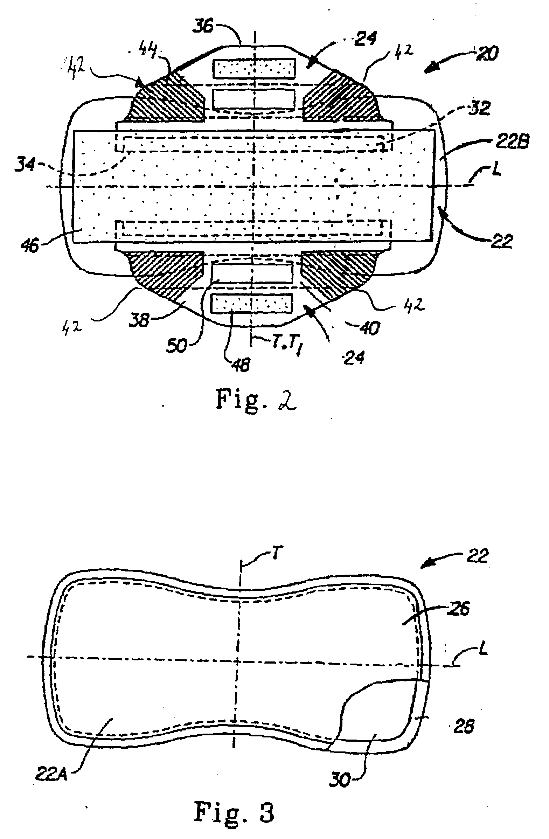

In another aspect of the present invention, the method of assembling web or film materials can be applied to the making of disposable absorbent articles. For the present invention, an example of a disposable absorbent sanitary napkin is described though the invention is not limited to such a configuration. The term "sanitary napkin", as used herein, refers to an article that is worn by females adjacent to the pudental region that is intended to absorb and contain the various exudates, which are discharged from the body (e.g., blood, menses and urine). As shown in Figure 2, the sanitary napkin 20 basically comprises an absorbent means represented by a central absorbent pad (or "main body portion") 22, and two flaps 24.

The sanitary napkin 20 has two centrelines, a principal longitudinal centreline L and a principal transverse centreline T. The term "longitudinal", as used herein, refers to a line, axis or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis or direction which lies within the plane of the sanitary napkin 20 that is generally perpendicular to the longitudinal direction.

The main body portion 22 of the sanitary napkin 20 has two surfaces, a liquid pervious body contacting surface or "body surface" 22A and a liquid impervious garment surface 22B. The sanitary napkin 20 is shown in Figure 2 as viewed from its garment surface 22B, and in Figure 3 as viewed from its body surface 22A. The body surface 22A is intended to be worn adjacent to the body of the wearer. The garment surface 22B of the main body portion 22 is intended to be placed adjacent to the undergarment when the sanitary napkin 20 is worn. The main body portion 22 of the sanitary napkin 20, as shown in Figure 3, generally comprises at least a topsheet 26, a backsheet 28, and an absorbent core 30. The topsheet 26, backsheet 28, and absorbent core 30 can comprise any of the materials generally used for these particular purposes. Suitable materials for these components, and preferred arrangements for the assembly of same are described in greater detail in US 5,389,094 and US 5,281,209.

The flaps 24 are each associated with the main body portion 22 along a juncture. This is typically a longitudinally-oriented (or "longitudinal") juncture, such as lines of juncture 32. As used herein, the terms "juncture" (or "line of juncture") refer to regions where the flaps 24 extend from or are joined to the main body portion 22. The flaps 24 can be integral with the main body portion 22, or with components of the main body portion 22, or they can comprise separate elements (i.e., pieces of material) that are joined to the main body portion 22. In the embodiment shown in Figure 2, the flaps 24 comprise separate pieces of material which are joined to the main body portion 22. The separate flap pieces are preferably joined to the main body portion 22 by applying adhesive to the flap pieces in the region that will form the line of juncture 32.

The flaps 24 have a proximal edge 34 adjacent to the line of juncture 32. A distal edge (or "free end") 36 is remote from the line of juncture 32. As shown in Figure 2, each flap 24 is divided into a front half 38, and a back half 40 by a flap transverse centerline T1. Preferably, as shown in Figure 2, the flaps 24 of the sanitary napkin 20 are provided with four zones of differential extensibility 42, one in the front half of each flap, and one in the back half of each flap. The term "zone of differential extensibility", as used herein, refers to a portion of the sanitary napkin 20 (in this case, the portion of the flaps 24) which is capable of extending a differing amount (preferably a greater amount), than surrounding portions of the sanitary napkin 20. The zones of differential extensibility 42 relieve the stresses that develop in the flaps 24 when they are folded around an undergarment crotch. The zone(s) of differential extensibility 42 can comprise any structure capable of extending a greater amount than the surrounding portions of the sanitary napkin. Suitable structures for the zones of differential extensibility 42 are described in US 5,389,094. In the preferred embodiment shown in Figure 2, the zones of differential extensibility 42 comprise regions of the flaps 24 that have been pre-corrugated or "ring rolled". The term "ring roll" refers to a process in which the material comprising the flaps is fed through intermeshing corrugated rolls. The flaps 24 can be ring rolled in accordance with methods described in US 4,107,364, US 4,834,741, US 5,143,679, US 5,156,793 and US 5,167,897. The ring rolling forms corrugations in the zones of differential extensibility 42. The corrugations are defined by fold lines or ridges and valleys 44. The fold lines 44 may form any angle desired relative to the principal longitudinal centreline L. In the preferred embodiment shown in Figure 2, the fold lines 44 form an angle of between about 40 degrees to 45 degrees with the principal longitudinal centreline L. This will provide the desired direction of extensibility.

Figure 2 also shows the fasteners, such as adhesive attachment means, central pad adhesive 46 and flap adhesive 48, which are adapted to secure the sanitary napkin 20 to the crotch region of an undergarment. The central pad adhesive 46 provides an adhesive attachment means for securing the main body portion 22 in the crotch portion of an undergarment. The garment facing surface of the flap 24 is preferably coated with a flap adhesive 48. The flap adhesive 48 can be positioned adjacent the distal edge 36 of the flap as shown. Alternatively, the positions of the flap adhesive 48 and the unitary release material 50 can be reversed. The flap adhesive 48 is used to assist in maintaining the flap 34 in position after it is wrapped around the edge of the crotch portion of the undergarment. The flaps 24 can be maintained in position by attaching the flaps 24 to the undergarment, or to the opposing flap. The central pad adhesive 46 is preferably covered with a removable release liner to keep the central pad adhesive 46 from sticking to extraneous surfaces prior to use. Preferably, the flaps 24 are provided with a unitary release material 50 that superposes the flap adhesive 48 when the flap 24 is folded along a fold line.

According to the teachings of the present invention, the first web or film material 10 supplied from the first supply assembly 12 may be the central pad adhesive 46 and the second web or film material 11 supplied from the second supply assembly 13 may be the backsheet 28. This example, however, is not limiting.

### GLOSSARY

- 10: First web or film material
- 11: Second web or film material
- 12: First supply assembly
- 13: Second supply assembly
- 14: Rotating drum/anvil roll
- 15: Knife roll
- 16: Transfer roll
- 17: Electrostatic charging unit
- 18: Guide rolls
- 19: Coating device
- 20: Sanitary napkin
- 22: Main body portion
- 22A: Liquid pervious body contacting surface
- 22B: Liquid impervious garment surface
- 24: Flaps
- 26: Topsheet
- 28: Backsheet
- 30: Absorbent core
- 32: Line of juncture
- 42: Zones of differential extensibility
- 44: Fold lines
- 46: Central pad adhesive
- 48: Flap adhesive
- 50: Unitary release material

## Claims

1. Method of assembling web or film materials during a continuous process, said method comprising the steps of:
(a) providing a first web or film material (10) from a first supply assembly (12);
(b) providing a second web or film material (11) from a second supply assembly (13);
(c) transferring said first web or film material (10) to said second web or film material (11) on a transfer roll (16);
characterised in that
before step (c), said method further comprises the step of:
imparting a static electrical charge to hold said first web or film material (10) in position on a rotating drum (14).

2. Method of assembling web or films according to claim 1 wherein said method further comprises the step of cutting said first web or film material (10) into pieces for application to said second web or film material (11) before imparting said static electrical charge.

3. Method of assembling web or film materials according to claim 1 wherein said method further comprises the step of cutting said first web or film material (10) into pieces for application to said second web or film material (11) after imparting said static electrical charge.

4. Method of assembling web or film materials according to any of the preceding claims wherein said static electrical charge is imparted to said first web or film material (10).

5. Method of assembling web or film materials according to any of the preceding claims wherein said static electrical charge imparted to said first web or film material (10) is opposite in polarity to the charge imparted to said second web or film material (11).

6. Method of assembling web or film materials according to claims 1, 2 and 3 wherein said static electrical charge is imparted to said rotating drum (14).

7. Method of assembling web or film materials according to any of the preceding claims wherein said first web or film material (10) is provided in the form of a coated layer of web or film material, preferably an adhesive coated layer of web or film material.

8. Method of assembling web or film materials according to any of the preceding claims wherein said first web or film material (10) is provided in the form of an uncoated layer of web or film material, preferably a non-adhesive coated layer of web or film material.

9. Method of assembling web or film materials according to any of the preceding claims wherein said first web or film material (10) and/or said second web or film material (11) are/is provided in the form of porous web or film materials.

10. Method of assembling web or film materials according to any of the preceding claims wherein said first web or film material (10) and/or said second web or film material (11) are/is discharged on unwinding from said first supply assembly (12) and/or said seconds supply assembly (13).

11. Method of assembling web or film materials according to any of the preceding claims wherein said method is applied to the making of disposable absorbent articles.
